# EUROPEAN PATENT APPLICATION

(11) **EP 4 729 027 A2**
(43) Date of publication of application: **22.04.2026**
(21) Application number: 25220051.4
(22) Date of filing: 10.02.2023
(51) Int. Cl.: A61F 2/24

(54) **LOCKING MECHANISM FOR A MECHANICALLY EXPANDABLE PROSTHETIC VALVE**

(30) Priority: 15.02.2022 US 202263268042 P
(62) Divisional of application: 23710538.2
(71) Applicant: Edwards Lifesciences Corporation, Irvine, CA 92614 (US)
(72) Inventor: Nir, Noam, Irvine, CA 92614 (US); Bukin, Michael, Irvine, CA 92614 (US)
(74) Representative: Eisenführ Speiser

(57) **Abstract**

An implantable device can include a radially expandable frame configured to move between a radially compressed state and a radially expanded state. The frame including a plurality of struts, at least one actuator mechanism configured to compress and expand the frame, and at least one locking mechanism coupled to the actuator mechanism. A first strut and a second strut can be pivotably coupled to one another at a common junction and configured to move toward one another as the frame is compressed and away from one another as the frame is expanded. The first and second struts can have first and second locking features that extend from respective longitudinal side edges of the struts. The locking mechanism can include a locking member configured to extend between and engage the first and second locking features when the frame is expanded to retain the frame in the radially expanded state.

## Description

### CROSS-REFERENCE TO RELATED APPLICATION

This application claims the benefit of U.S. Provisional Application No. 63/268,042, filed February 15, 2022, which is incorporated herein by reference.

### FIELD

The present disclosure relates to implantable, mechanically expandable prosthetic devices, such as prosthetic heart valves, and to methods and delivery assemblies for, and including, such prosthetic devices.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (such as stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery apparatus and advanced through the patient's vasculature (such as through a femoral artery and the aorta) until the prosthetic heart valve reaches the implantation site in the heart. The prosthetic heart valve is then expanded to its functional size, for example, by inflating a balloon on which the prosthetic valve is mounted, actuating a mechanical actuator that applies an expansion force to the prosthetic heart valve, or by deploying the prosthetic heart valve from a sheath of the delivery apparatus so that the prosthetic heart valve can self-expand to its functional size.

Prosthetic heart valves that rely on a mechanical actuator for expansion can be referred to as "mechanically expandable" prosthetic heart valves. Mechanically expandable prosthetic heart valves can provide one or more advantages over self-expandable and balloon-expandable prosthetic heart valves. For example, mechanically expandable prosthetic heart valves can be expanded to various working diameters. Mechanically expandable prosthetic heart valves can also be compressed after an initial expansion (such as for repositioning and/or retrieval).

Despite the recent advancements in percutaneous valve technology, there remains a need for improved transcatheter heart valves and delivery devices for such prosthetic valves.

### SUMMARY

Described herein are prosthetic heart valves, delivery apparatus, and methods for implanting prosthetic heart valves. The disclosed prosthetic heart valves, delivery apparatus, and methods can, for example, provide for repositionable prosthetic heart valves. As such, the devices and methods disclosed herein can, among other things, overcome one or more of the deficiencies of typical prosthetic heart valves and their delivery apparatus.

An implantable medical device (such as a prosthetic heart valve) can comprise a radially expandable frame comprising a plurality of struts. In addition to these components, an implantable medical device can further comprise one or more of the components disclosed herein.

In some examples, an implantable medical device can comprise at least one actuator mechanism configured to radially compress and expand the frame.

In some examples, the plurality of struts can include first and second struts pivotably coupled to each other at a common junction, wherein the first and second struts can have first and second locking features that extend from longitudinal side edges of the first and second struts, respectively.

In some examples, an implantable medical device can comprise at least one locking mechanism configured to retain the frame in a radially expanded state.

In some examples, the locking mechanism can comprise a locking member configured to extend between and engage the first and second locking features when the frame is expanded to retain the frame in a radially expanded state.

In some examples, the locking mechanism can comprise a wedge member.

In some examples, the locking member can be configured to move between a locked position in which the locking member extends between and engages the first and second locking features and an unlocked position in which the locking member allows the first strut and the second strut to move toward one another and the frame to move from the expanded state into the compressed state.

In some examples, the first and second locking features can each comprise one or more teeth configured to engage the locking member in the locked position.

In some examples, the teeth can extend in the circumferential direction.

In some examples, the teeth of the first locking feature can be integrally formed on the first strut and the teeth of the second locking feature can be integrally formed on the second strut.

In some examples, the first and second locking features can each comprise a rounded edge which curves radially outwardly from a longitudinal side edge of a respective one of the first and second struts.

In some examples, an implantable medical device can comprise a release member releasably coupled to the locking member and configured to move the locking member from the locked position to the unlocked position.

In some examples, the locking mechanism can comprise a biasing member configured to bias the locking member in a first direction to engage the first and second locking features.

In some examples, the release member can be configured to retract the locking member in a second direction opposite the first direction of the bias of the locking member to move the locking member to the unlocked position.

In some examples, the locking member and the biasing member can be coaxially aligned.

In some examples, the locking member can be configured to extend between and engage the first and second locking features when the frame is in a first radially expanded state and when the frame is in a second radially expanded state.

In some examples, the frame can have a first diameter in the first radially expanded state and a second diameter in the second radially expanded state.

In some examples, the second diameter of the frame can be greater than the first diameter of the frame.

In some examples, the locking mechanism can be configured to retain the frame in two or more successive radially expanded states.

In some examples, a diameter of the frame can progressively increase with each successive radially expanded state.

In some examples, the locking mechanism can move progressively closer to the common junction with each successive radially expanded state.

In some examples, the locking member can extend radially from an inner surface of the frame to an outer surface of the frame.

In some examples, the first and second locking features can be directed in opposing circumferentially extending directions.

In some examples, the first and second locking features can be located proximate the common junction of the first and second struts.

In some examples, the first and second locking features can be located in an outflow region of the frame.

In some examples, the first and second locking features can be integrally formed with their respective struts.

In some examples, at least one actuator mechanism can comprise a plurality of actuator mechanisms and the at least one locking mechanism can comprise a plurality of locking mechanisms, each locking mechanism being coupled to a respective actuator mechanism.

In some examples, an implantable device can comprise a radially expandable frame configured to move between a radially compressed state and a radially expanded state. The frame can comprise a plurality of struts, at least one actuator mechanism configured to compress and expand the frame, and at least one locking mechanism coupled to the actuator mechanism. A first strut and a second strut of the plurality of struts can be pivotably coupled to one another at a common junction and configured to move toward one another as the frame is compressed and away from one another as the frame is expanded. The first and second struts can have first and second locking features that extend from longitudinal side edges of the first and second struts, respectively. The locking mechanism can comprise a locking member configured to extend between and engage the first and second locking features when the frame is expanded to retain the frame in the radially expanded state.

In some examples, an implantable device can comprise a radially expandable frame configured to move between a radially compressed state and a radially expanded state. The frame can comprise a plurality of inner and outer struts forming an inflow end and an outflow end of the frame, an inner strut forming an inner circumferentially extending locking feature and an outer strut pivotably coupled to and adjacent the inner strut forming an outer circumferentially extending locking feature. The frame can also comprise at least one expansion mechanism configured to radially compress and expand the frame, and at least one locking mechanism coupled to the expansion mechanism and comprising a locking member configured to engage the inner and outer locking features of the inner and outer struts. When the frame is in the radially compressed state, the inner and outer locking features can overlap in a radial direction, and when the frame is in the radially expanded state, the inner and outer locking features can be spaced circumferentially from one another and the locking member of the locking mechanism can extend between and engage the inner and outer locking features to retain the frame in the radially expanded state.

In some examples, an implantable device can comprise a radially expandable frame configured to move between a radially compressed state and a radially expanded state. The frame can comprise a plurality of inner and outer struts forming an inflow end and an outflow end, an inner strut having an inner locking feature circumferentially extending from a longitudinal side edge of the inner strut, and an outer strut pivotably coupled to and adjacent the inner strut having an outer locking feature circumferentially extending from a longitudinal side edge of the outer strut. The frame can also comprise at least one expansion mechanism configured to compress and expand the frame, and a locking mechanism coupled to the expansion mechanism and comprising a wedge member configured to engage the inner and outer locking features and a biasing member configured to bias the wedge member between the inner and outer locking features as the frame is radially expanded. The inner and outer struts can be configured to move toward one another and overlap as the frame is radially compressed and move away from one another and circumferentially separate as the frame is radially expanded. The wedge member can engage an end portion of at least one of the inner and outer locking features when the frame is in the radially compressed state, and extend and wedge between the inner and outer locking features as the frame is radially expanded such that the inner and outer struts and frame are retained in the radially expanded state.

In some examples, a method can comprise inserting a distal end of a delivery apparatus into a vasculature of a patient, wherein the delivery apparatus can be releasably coupled to a prosthetic heart valve comprising a radially expandable frame configured to move between a radially compressed state and a radially expanded state. The frame can comprise a plurality of struts, at least one expansion mechanism configured to compress and expand the frame, and at least one locking mechanism coupled to the expansion mechanism and comprising a locking member. A first strut can comprise a first circumferentially extending locking feature and a second strut coupled to and adjacent the first strut can comprise a second circumferentially extending locking feature. The method can further comprise advancing the prosthetic heart valve to a selected implantation location, expanding the frame of the prosthetic heart valve, and wedging the locking member of the locking mechanism between the first and second locking features. Engagement between the locking member and the first and second locking features can prevent the struts from moving in a first direction to compress the frame and permits the strut to move in a second direction to radially expand the frame.

The above method(s) can be performed on a living animal or on a simulation, such as on a cadaver, cadaver heart, anthropomorphic ghost, simulator (e.g., with body parts, heart, tissue, etc. being simulated).

In some examples, an implantable medical device comprises one or more of the components recited in Examples 1-53 below.

The various innovations of this disclosure can be used in combination or separately. This summary is provided to introduce a selection of concepts in a simplified form that are further described below in the detailed description. This summary is not intended to identify key features or essential features of the claimed subject matter, nor is it intended to be used to limit the scope of the claimed subject matter. The foregoing and other objects, features, and advantages of the disclosure will become more apparent from the following detailed description, claims, and accompanying figures.

### BRIEF DESCRIPTION OF THE DRAWINGS

FIG. 1 is a perspective view of a prosthetic heart valve and corresponding frame, according to one example.
FIG. 2A is a side elevation view of the frame of the prosthetic heart valve of FIG. 1, shown in a radially compressed state.
FIG. 2B is a side elevation view of the frame of the prosthetic heart valve of FIG. 1, shown in a radially expanded state.
FIG. 3 is a side view of a frame for a prosthetic heart valve according to another example.
FIG. 4 is a magnified view of a locking mechanism and a pair of locking features of the frame of FIG. 3.
FIG. 5 is a side view of the locking mechanism and struts of FIG. 4.
FIG. 6 is a magnified view of a different example of a locking mechanism shown engaging a pair of locking features of the frame of FIG. 3.
FIG. 7 is a magnified view of the locking mechanism of FIG. 6 engaging a pair of locking features of a frame, wherein the locking features have a different configuration than those shown in FIG. 6.
FIG. 8 is a magnified view of a locking mechanism according to another example.
FIG. 9 is a side elevation view of a delivery apparatus for a prosthetic heart valve, according one example.

### DETAILED DESCRIPTION

### General Considerations

For purposes of this description, certain aspects, advantages, and novel features of the examples of this disclosure are described herein. The disclosed methods, apparatus, and systems should not be construed as being limiting in any way. Instead, the present disclosure is directed toward all novel and nonobvious features and aspects of the various disclosed examples, alone and in various combinations and sub-combinations with one another. The methods, apparatus, and systems are not limited to any specific aspect or feature or combination thereof, nor do the disclosed examples require that any one or more specific advantages be present or problems be solved.

Although the operations of some of the disclosed examples are described in a particular, sequential order for convenient presentation, it should be understood that this manner of description encompasses rearrangement, unless particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used in this application and in the claims, the singular forms "a," "an," and "the" include the plural forms unless the context clearly dictates otherwise. Additionally, the term "includes" means "comprises." Further, the term "coupled" and "connected" generally mean physically, mechanically, chemically, magnetically, and/or electrically coupled or linked and does not excluded the presence of intermediate elements between the coupled or associated items absent specific contrary language. As used herein, "and/or" means "and" or "or", as well as "and" and "or".

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (such as out of the patient's body), while the distal motion of the device is motion of the device away from the user and toward the implantation site (such as into the patient's body). The terms "longitudinal" and "axial" refer to an axis extending in the proximal and distal directions, unless otherwise expressly defined.

It should be understood that the disclosed examples can be adapted to deliver and implant prosthetic devices in any of the native annuluses of the heart (such as the aortic, pulmonary, mitral, and tricuspid annuluses), and can be used with any of various delivery approaches (such as retrograde, antegrade, transseptal, transventricular, transatrial, etc.).

As used herein, "e.g." means "for example," and "i.e." means "that is."

### Examples of the Disclosed Technology

Described herein are examples of frames for use with such prosthetic implants. The frames can comprise expansion and locking mechanisms configured to expand and/or compress the frame and to retain the frame in an expanded configuration when the implant is expanded at a selected delivery site within a patient. The frames can improve a physician's ability to control the size of a mechanically-expandable prosthetic implant, such as prosthetic valves (such as prosthetic heart valves or venous valves), stents, or grafts.

Prosthetic valves disclosed herein can be radially compressible and expandable between a radially compressed state and a radially expanded state. Thus, the prosthetic valves can be crimped on or retained by an implant delivery apparatus in the radially compressed state during delivery, and then expanded to the radially expanded state once the prosthetic valve reaches the implantation site. It is understood that the valves disclosed herein may be used with a variety of implant delivery apparatuses, and examples thereof will be discussed in more detail later.

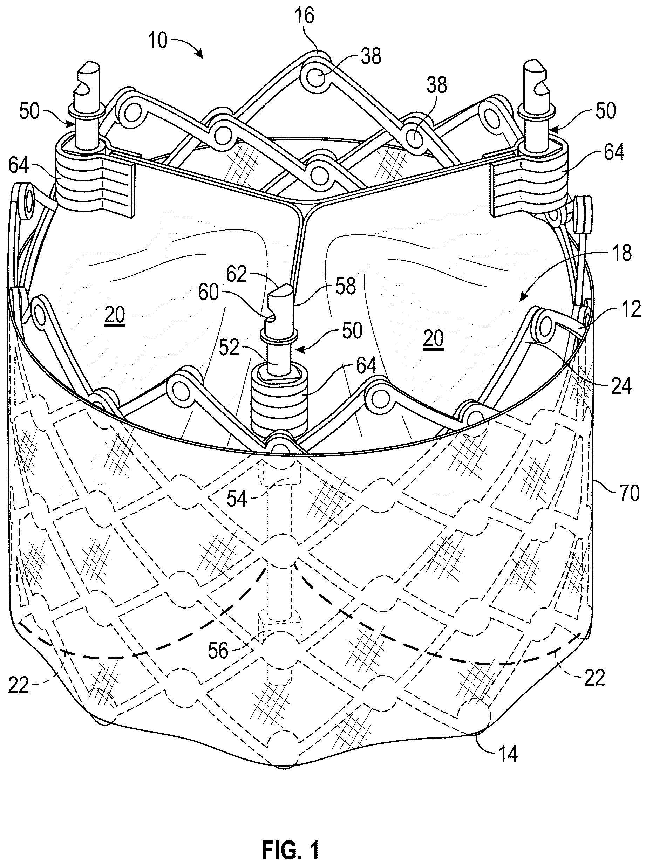

FIG. 1 shows an exemplary prosthetic valve 10, according to one example. Any of the prosthetic valves disclosed herein are adapted to be implanted in the native aortic annulus, although in other examples they can be adapted to be implanted in the other native annuluses of the heart (the pulmonary, mitral, and tricuspid valves). The disclosed prosthetic valves also can be implanted within vessels communicating with the heart, including a pulmonary artery (for replacing the function of a diseased pulmonary valve, or the superior vena cava or the inferior vena cava (for replacing the function of a diseased tricuspid valve) or various other veins, arteries and vessels of a patient. The disclosed prosthetic valves also can be implanted within a previously implanted prosthetic valve (which can be a prosthetic surgical valve or a prosthetic transcatheter heart valve) in a valve-in-valve procedure.

In some examples, the disclosed prosthetic valves can be implanted within a docking or anchoring device that is implanted within a native heart valve or a vessel. For example, in one example, the disclosed prosthetic valves can be implanted within a docking device implanted within the pulmonary artery for replacing the function of a diseased pulmonary valve, such as disclosed in U.S. Publication No. 2017/0231756, which is incorporated by reference herein.

In some examples, the disclosed prosthetic valves can be implanted within a docking device implanted within or at the native mitral valve, such as disclosed in PCT Publication No. WO2020/247907, which is incorporated herein by reference.

In some examples, the disclosed prosthetic valves can be implanted within a docking device implanted within the superior or inferior vena cava for replacing the function of a diseased tricuspid valve, such as disclosed in U.S. Publication No. 2019/0000615, which is incorporated herein by reference.

The prosthetic valve 10 can include an annular stent or frame 12 having an inflow end 14 and an outflow end 16. The prosthetic valve 10 can also include a valvular structure 18 which is coupled to and supported inside of the frame 12. The valvular structure 18 is configured to regulate the flow of blood through the prosthetic valve 10 from the inflow end 14 to the outflow end 16.

The valvular structure 18 can include, for example, a leaflet assembly comprising one or more leaflets 20 made of a flexible material. The leaflets 20 can be made from in whole or part, biological material, bio-compatible synthetic materials, or other such materials. Suitable biological material can include, for example, bovine pericardium (or pericardium from other sources). The leaflets 20 can be secured to one another at their adjacent sides to form commissures, each of which can be secured to a respective actuator 50 or the frame 12.

In some examples, the valvular structure 18 comprises three leaflets 20, which can be arranged to collapse in a tricuspid arrangement. Each leaflet 20 can have an inflow edge portion 22. As shown in FIG. 1, the inflow edge portions 22 of the leaflets 20 can define an undulating, curved scallop shape that follows or tracks a plurality of interconnected strut segments of the frame 12 in a circumferential direction when the frame 12 is in the radially expanded configuration. The inflow edges of the leaflets can be referred to as a "scallop line."

In some examples, the inflow edge portions 22 of the leaflets 20 can be sutured to adjacent struts of the frame generally along the scallop line.

In some examples, the inflow edge portions 22 of the leaflets 20 can be sutured to an inner skirt, which in turn in sutured to adjacent struts of the frame. By forming the leaflets 20 with this scallop geometry, stresses on the leaflets 20 are reduced, which in turn improves durability of the valve 10. Moreover, by virtue of the scallop shape, folds and ripples at the belly of each leaflet 20 (the central region of each leaflet), which can cause early calcification in those areas, can be eliminated or at least minimized. The scallop geometry also reduces the amount of tissue material used to form valvular structure 18, thereby allowing a smaller, more even crimped profile at the inflow end 14 of the valve 10.

Further details regarding transcatheter prosthetic heart valves, including the manner in which the valvular structure can be mounted to the frame of the prosthetic valve can be found, for example, in U.S. Patent Nos. 6,730,118, 7,393,360, 7,510,575, 7,993,394, and 8,252,202, U.S. Patent Application No. 15/978,459 (Published as U.S. Publication No. 2018/0325665), U.S. Provisional Application No. 62/854,702, filed May 30, 2019, and U.S. Patent Application No. 16/941,776, filed July 29, 2020, all of which are incorporated herein by reference in their entireties.

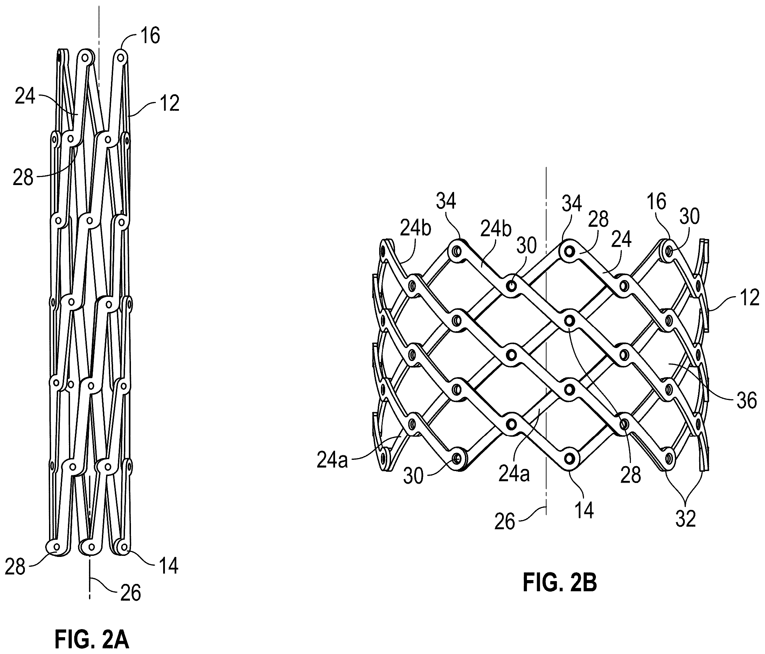

The prosthetic valve 10 can be radially compressible and expandable between a radially compressed configuration and a radially expanded configuration. FIGS. 2A-2B show the bare frame 12 of the prosthetic valve 10 (without the leaflets and other components) for purposes of illustrating expansion of the prosthetic valve 10 from the radially compressed configuration (FIG. 2A) to the radially expanded configuration (FIG. 2B).

The frame 12 can include a plurality of interconnected lattice struts 24 arranged in a lattice-type pattern and forming a plurality of apices 34 at the outflow end 16 of the prosthetic valve 10. The struts 24 can also form similar apices 32 at the inflow end 14 of the prosthetic valve 10. As shown in FIG. 2B, the struts 24 are shown as positioned diagonally, or offset at an angle relative to, and radially offset from, a longitudinal axis 26 of the prosthetic valve 10 when the prosthetic valve 10 is in the expanded configuration.

In some examples, the struts 24 can be offset by a different amount than depicted in FIG. 2B, or some or all of the struts 24 can be positioned parallel to the longitudinal axis 26 of the prosthetic valve 10.

The struts 24 can comprise a set of inner struts 24a (extending from the lower left to the upper right of the frame in FIG. 2B) and a set of outer struts 24b (extending from the upper left to the lower right of the frame in FIG. 2B) connected to the inner struts 24a. The open lattice structure of the frame 12 can define a plurality of open frame cells 36 between the struts 24.

The struts 24 can be pivotably coupled to one another at one or more pivot joints or pivot junctions 28 along the length of each strut. For instance, in one example, each of the struts 24 can be formed with apertures 30 at opposing ends of the strut and apertures spaced along the length of the strut. Respective hinges can be formed at the locations where the inner and outer struts 24a-24b overlap each other via fasteners 38 (FIG. 1), such as rivets or pins that extend through the apertures 30. The hinges can allow the struts 24 to pivot relative to one another as the frame 12 is radially expanded or compressed, such as during assembly, preparation, or implantation of the prosthetic valve 10.

The frame struts and the components used to form the pivot joints of the frame 12 (or any frames described below) can be made of any of various suitable materials, such as stainless steel, a cobalt chromium alloy, or a nickel titanium alloy ("NiTi"), for example Nitinol. In some examples, the frame 12 can be constructed by forming individual components (such as the struts and fasteners of the frame) and then mechanically assembling and connecting the individual components together. Further details regarding the construction of the frame and the prosthetic valve are described in U.S. Patent Nos. 10,603,165 and 10,806,573, U.S. Publication Nos. 2018/0344456 and 2020/0188099, all of which are incorporated herein by reference.

In some examples, the prosthetic valve 10 can be mechanically expanded from the radially contracted configuration to the radially expanded configuration. For instance, the prosthetic valve 10 can be radially expanded by maintaining the inflow end 14 of the frame 12 at a fixed position while applying a force in the axial direction against the outflow end 16 toward the inflow end 14.

In some examples, the prosthetic valve 10 can be expanded by applying an axial force against the inflow end 14 while maintaining the outflow end 16 at a fixed position, or by applying opposing axial forces to the inflow and outflow ends 14, 16, respectively.

As shown in FIG. 1, the prosthetic valve 10 can include one or more actuators 50 mounted to and equally spaced around the inner surface of the frame 12. Each of the actuators 50 can be configured to form a releasable connection with one or more respective actuators of a delivery apparatus.

In some examples, expansion and compression forces can be applied to the frame by the actuators 50. Referring again to FIG. 1, each of the actuators 50 can comprise a screw or threaded rod 52, a first anchor in the form of a cylinder or sleeve 54, and a second anchor in the form of a threaded nut 56. The rod 52 extends through the sleeve 54 and the nut 56. The sleeve 54 can be secured to the frame 12, such as with a fastener 38 that forms a hinge at the junction between two struts. Each actuator 50 is configured to increase the distance between the attachment locations of a respective sleeve 54 and nut 56, which causes the frame 12 to elongate axially and compress radially, and to decrease the distance between the attachment locations of a respective sleeve 54 and nut 56, which causes the frame 12 to foreshorten axially and expand radially.

In some examples, each rod 52 can have external threads that engage internal threads of the nut 56 such that rotation of the rod causes corresponding axial movement of the nut 56 toward or away from the sleeve 54 (depending on the direction of rotation of the rod 52). This causes the hinges supporting the sleeve 54 and the nut 56 to move closer towards each other to radially expand the frame or to move farther away from each other to radially compress the frame, depending on the direction of rotation of the rod 52.

In some examples, the actuators 50 can be reciprocating type actuators configured to apply axial directed forces to the frame to produce radial expansion and compression of the frame. For instance, the rod 52 of each actuator can be fixed axially relative to the nut 56 and slidable relative to the sleeve 54. Thus, in this manner, moving the rod 52 distally relative to the sleeve 54 and/or moving the sleeve 54 proximally relative to the rod 52 radially compresses the frame. Conversely, moving the rod 52 proximally relative to the sleeve 54 and/or moving the sleeve 54 distally relative to the rod 52 radially expands the frame.

When reciprocating type actuators are used, the prosthetic valve can also include one or more locking mechanisms that retain the frame in the expanded state. The locking mechanisms can be separate components that are mounted on the frame apart from the actuators, or they can be a sub-component of the actuators themselves.

Each rod 52 can include an attachment member 58 along a proximal end portion of the rod 52 configured to form a releasable connection with a corresponding actuator of a delivery apparatus. The actuator(s) of the delivery apparatus can apply forces to the rods for radially compressing or expanding the prosthetic valve 10. The attachment member 58 in the illustrated configuration comprises a notch 60 and a projection 62 that can engage a corresponding projection of an actuator of the delivery apparatus.

In some examples, the prosthetic valve 10 includes three such actuators 50, although a greater or fewer number of actuators could be used in other examples. The leaflets 20 can have commissure attachments members 64 that wrap around the sleeves 54 of the actuators 50. Further details of the actuators, locking mechanisms and delivery apparatuses for actuating the actuators can be found in U.S. Patent No. 10,603,165 and U.S. Publication Nos. 2019/0060057, and 2018/0325665, each of which is incorporated herein by reference in its entirety. Any of the actuators and locking mechanisms disclosed in the previously filed applications can be incorporated in any of the prosthetic valves and frames disclosed herein. Further, any of the delivery apparatuses disclosed in the previously filed applications can be used to deliver and implant any of the prosthetic valves disclosed herein.

The prosthetic valve 10 can include one or more skirts or sealing members. In some examples, the prosthetic valve 10 can include an inner skirt (not shown) mounted on the inner surface of the frame. The inner skirt can function as a sealing member to prevent or decrease perivalvular leakage, to anchor the leaflets to the frame, and/or to protect the leaflets against damage caused by contact with the frame during crimping and during working cycles of the prosthetic valve. As shown in FIG. 1, the prosthetic valve 10 can also include an outer skirt 70 mounted on the outer surface of the frame 12. The outer skirt 70 can function as a sealing member for the prosthetic valve by sealing against the tissue of the native valve annulus and helping to reduce paravalvular leakage past the prosthetic valve. The inner and outer skirts can be formed from any of various suitable biocompatible materials, including any of various synthetic materials, including fabrics (such as polyethylene terephthalate fabric) or natural tissue (such as pericardial tissue). Further details regarding the use of skirts or sealing members in prosthetic valve can be found, for example, in U.S. Patent No. 10,806,573, which is incorporated herein by reference in its entirety.

The skirts can be wholly or partly formed of any suitable biological material, synthetic material (for example, any of various polymers), or combinations thereof. In some examples, the skirts can comprise a fabric having interlaced yarns or fibers, such as in the form of a woven, braided, or knitted fabric. In some examples, the fabric can have a plush nap or pile. Exemplary fabrics having a plus nap or pile include velour, velvet, velveteen, corduroy, terrycloth, fleece, etc. In some examples, the skirts can comprise a fabric without interlaced yarns or fibers, such as felt or an electrospun fabric. Exemplary materials that can be used for forming such fabrics (with or without interlaced yarns or fibers) include, without limitation, polyethylene (PET), ultrahigh molecular weight polyethylene (UHMWPE), polytetrafluoroethylene (PTFE), expanded polytetrafluoroethylene (ePTFE), polyamide etc. In some examples, the skirts can comprise a non-textile or non-fabric material, such as a film including any of a variety of polymeric materials, such as PTFE, PET, polypropylene, polyamide, polyetheretherketone (PEEK), polyurethane (such as thermoplastic polyurethane (TPU)), etc. In some examples, the skirts can comprise a sponge material or foam, such as polyurethane foam. In some examples, the skirts can comprise natural tissue, such as pericardium (for example, bovine pericardium, porcine pericardium, equine pericardium, or pericardium from other sources).

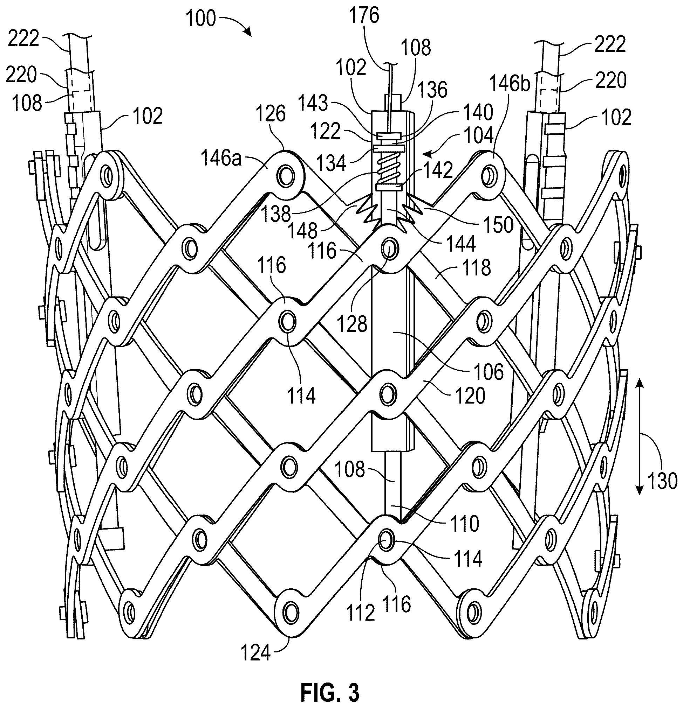

FIG. 3 illustrates another example of a frame 100 in a radially expanded configuration, which can be used in a prosthetic valve. The frame 100 can include all of the components as previously described in connection with FIGS. 1-2B and frame 12, a detailed description of which is not repeated here in the interest of brevity. To form a prosthetic valve, it should be understood that the frame 100 can also include the leaflets 20 and other soft components (such as the outer skirt 70) described in connection with the prosthetic valve 10, which have not been included for the purposes of illustration. While only on side of the frame 100 is depicted in FIG. 3, it should be appreciated that the frame 100 forms an annular structure.

As shown in FIG. 3, the frame 100 can comprise one or more expansion mechanisms 102 (also referred to as "actuators") and locking mechanisms 104. Each expansion mechanism 102 can have a respective locking mechanism 104 coupled thereto. Although FIG. 3 shows three expansion mechanisms 102 mounted to the frame 100, it should be appreciated that the frame 100 can comprise any number of expansion mechanisms 102 and locking mechanisms 104. For instance, in some examples, a prosthetic valve comprising the frame 100 can include a single expansion mechanism and a respective locking mechanism, two expansion mechanisms and two locking mechanisms, or four or more expansion mechanisms and four or more corresponding locking mechanisms. The expansion mechanisms 102 can be placed at any position about the circumference of the frame 100. For instance, each pair of an expansion mechanism 102 and a locking mechanism 104 can be equally spaced from one adjacent pair about a circumference of the frame 100.

Each expansion mechanism 102 can include a first or outer member 106 and a second or inner member 108. The outer member 106 can comprise a first lumen or bore sized to receive at least a portion of the inner member 108. The outer and inner members 106, 108 can be coupled and configured to move in a telescoping manner, axially relative one another, to radially expand and compress the frame 100.

A distal end portion 110 of the inner member 108 can be coupled to the frame 100 at a first location via a fastener 112 that is affixed to and extends radially from the distal end portion 110 of the inner member 108. The fastener 112 can be, for instance, a rivet or a pin which extends through corresponding apertures 114 in overlapping inner and outer struts 118, 120 at a junction 116 of the struts. The fastener 112 can serve as a pivot pin about which the two struts 118, 120 can pivot relative to one another and the inner member 108. In some examples, an end cap or nut can be disposed over an end portion of the fastener 112 to retain the fastener within the corresponding apertures.

The outer member 106 can be similarly coupled to the frame 100 at a second location, which is axially spaced from the first location where the inner member 108 is coupled to the frame. In particular, the inner member 108 can be secured to the frame 100 proximate an inflow end 124 of the frame and a proximal end portion 122 of the outer member 106 can be secured proximate an outflow end 126 of the frame. The outer member 106 can be secured to the frame 100 via a fastener 128, which can also a rivet or a pin. The fastener 128 can also be affixed to and extend radially from the outer member 106 through corresponding apertures 114 at a junction 116 formed of overlapping inner and outer struts 118, 120 and serve as a pivot pin about which the two struts 118, 120 pivot relative to each other and the outer member 106. A nut can be mounted on each fastener 128 to retain the fastener within the corresponding apertures. In another configuration, the outer and inner members 106, 108 can be similarly coupled to the frame 100 at any two axially spaced and circumferentially aligned locations on the frame. In some examples, the outer and inner members 106, 108 need not comprise fasteners 112, 128 and can be coupled to the frame 100 via other forms of attachment, such as welding, adhesives, etc.

As previously mentioned, the inner member 108 can be axially movable relative to the outer member 106. As indicated by arrow 130 in FIG. 3, for example, the outer and inner members 106, 108 can move relative to one another in an axial direction toward the outflow end 126 and/or the inflow end 124 of the frame 100. Since the inner member 108 and the outer member 106 are secured to the frame 100 at axially spaced locations, moving the outer and inner members 106, 108 axially relative to one another in a telescoping manner can cause radial expansion and/or compression of the frame 100. Specifically, moving the inner member 108 axially toward the outflow end 126 of the frame 100 while holding the outer member 106 in a fixed position and/or moving the outer member 106 axially toward the inflow end 124 of the frame 100, can cause the frame 100 to foreshorten axially and expand radially. Conversely, moving the inner member 108 axially in the direction of the inflow end 124 and/or moving the outer member 106 toward the outflow end 126 of the frame 100, can cause the frame 100 to elongate axially and compress radially. This telescoping movement of outer and inner members 106, 108 relative to one another can advantageously provide continuous valve expansion wherein the expansion mechanism 102 can be easily maneuvered between the locked and unlocked configurations.

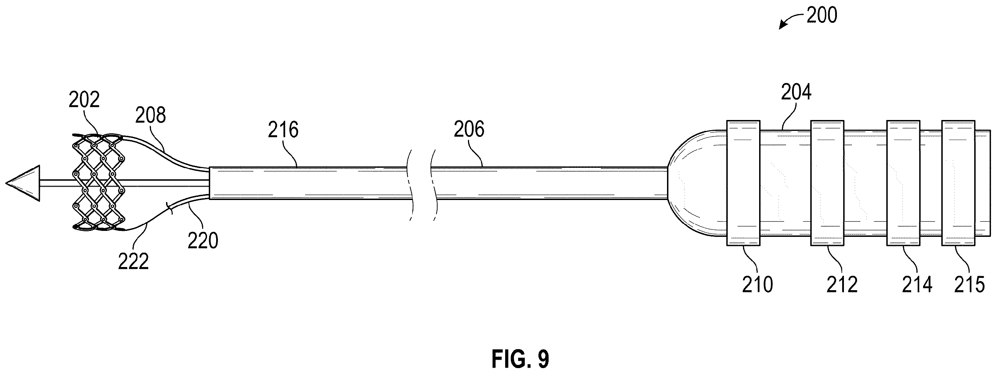

The expansion mechanisms 102 can be releasably coupled to an actuator assembly of a delivery apparatus for actuating the expansion mechanisms 102 for radially expanding and/or compressing the frame 100, as further described below. For example, each expansion mechanism 102 can be releasably coupled to an actuator assembly 208 of a delivery apparatus 200 (see FIG. 9). As described in greater detail below, each actuator assembly 208 can include an outer sleeve member 220 having a distal end that can abut and push against the proximal end of the outer member 106 and an inner actuator member 222 that can be releasably connected to the inner member 108 while the prosthetic valve is being implanted within a patient. The outer sleeve member 220 and the inner actuator member 222 can be moved axially relative to each other to produce corresponding axial movement of the outer member 106 and the inner member 108 relative to each other for radially expanding and/or compressing the frame 100.

Again, it will be appreciated that alternate expansion mechanisms can be coupled to the frame 100 to effect radial expansion and compression of the frame. Further details of other types of expansion mechanisms can be found, for example, in U.S. Patent No. 10,603,165. Any of the expansion mechanisms disclosed in U.S. Patent No. 10,603,165 can be implemented in the frame 100.

During implantation, a prosthetic valve comprising the frame 100 can be placed in a radially compressed or collapsed state and inserted into a patient using a delivery apparatus (such as delivery apparatus 200). Once positioned in a desired implantation site, the frame 100 can be placed in a radially expanded and operational state. For instance, to deploy the frame 100, an operator can actuate the delivery apparatus (such as the actuator assemblies 208) and thereby actuate the one or more expansion mechanisms 102. As indicated by the arrow 130 in FIG. 3, the inner member 108 can move toward the outflow end 126 and/or the outer member 106 can move toward the inflow end 124 to decrease the distance between the attachment locations of the outer and inner members 106, 108. This causes the frame 100 to foreshorten axially and expand radially until a desired diameter is achieved. As the frame 100 is radially expanded, the frame 100 can be locked or retained in the desired expanded state via the locking mechanisms 104.

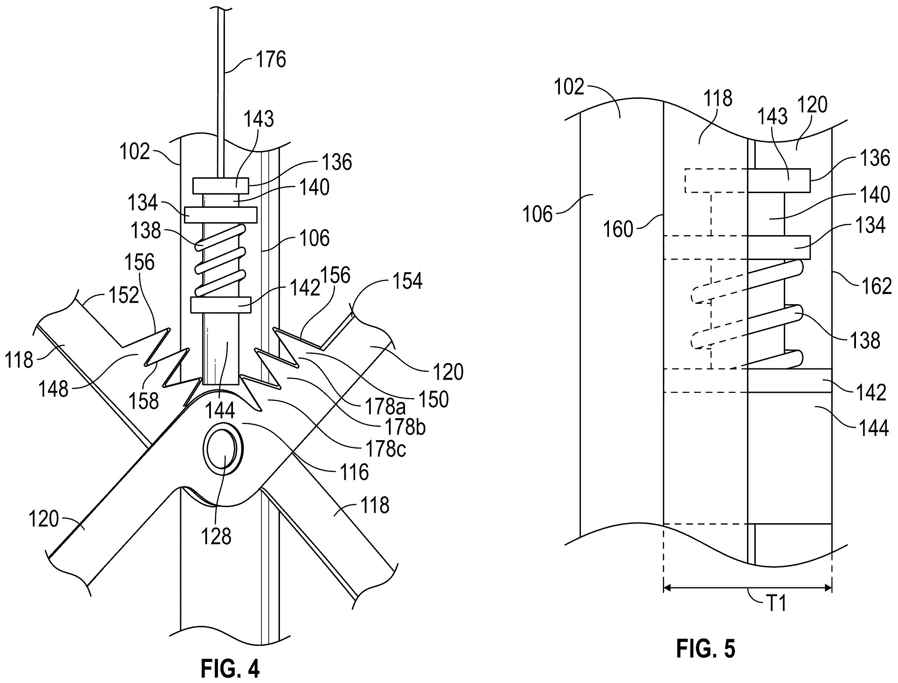

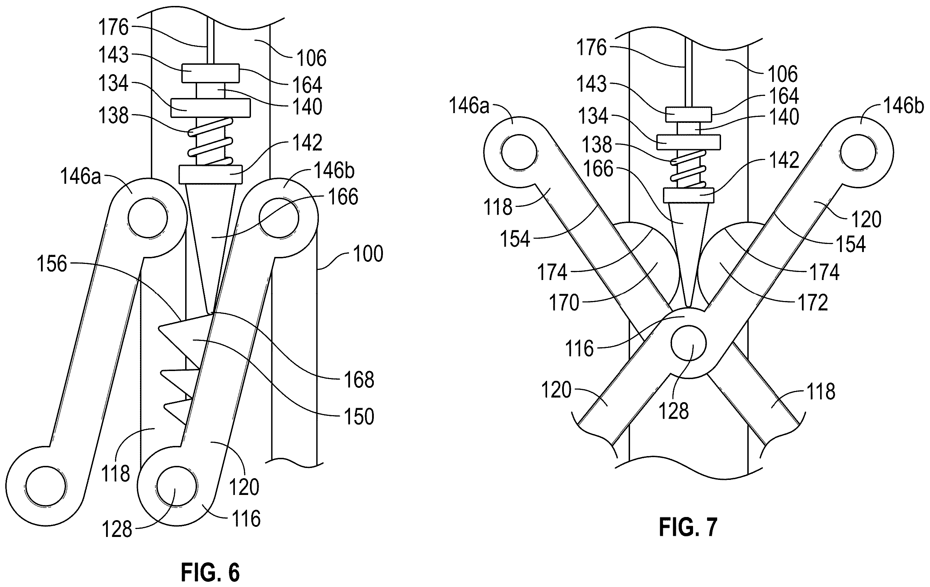

Referring to FIGS. 3-5, each locking mechanism 104 can comprise a support member 134, a locking member 136 coupled to the support member 134, and a biasing member 138. The support member 134 can be a cantilevered support coupled to and extending perpendicularly and outwardly from a respective expansion mechanism 102. The support member 134 can generally position the locking member 136 between a pair of adjacent inner and outer struts 118, 120 when the frame 100 is both in a radially compressed state and a radially expanded state. For example, the locking member 136 can extend at least partially between respective inner and outer struts 118, 120 when the frame 100 is in a compressed (see FIG. 6) and an expanded state see FIGS. 4 and 7). This configuration also positions the locking member 136 parallel to a respective expansion mechanism 102, each of which can be parallel to a longitudinal axis of the frame 100 (such as the longitudinal axis 26).

A bore (not shown) extending through the support member 134 can be configured to retain and allow the locking member 136 to move axially relative to the support member 134 and expansion mechanism 102. For instance, the locking member 136 can comprise a pin-like body 140 which freely extends through the bore of the support member 134, permitting the locking member 136 to move axially alongside a respective expansion mechanism 102, such as in a longitudinal direction toward the inflow end 124 and/or the outflow end 126 of the frame 100. By extension, the locking member 136 is also configured to move axially relative to respective inner and outer struts 118, 120 the locking member 136 is situated between.

The locking member 136 in the illustrated example can also comprise at one end of its body 140 a collar or flange 142 and an engagement or wedge member 144 axially extending from the flange 142. The locking member 136 can further include another collar or flange 143 at the proximal end of the body 140 to prevent the locking member from sliding distally past the support member 134. As illustrated in FIGS. 4 and 5, the biasing member 138 (such as a compression spring in the illustrated example) of the locking mechanism 104 can be situated between the flange 142 and support member 134. The biasing member 138 can be movable between a compressed configuration and an extended configuration as the body 140 of the locking member 136 moves relative to the support member 134. For instance, as the body 140 and flange 142 move axially toward the support member 134 and outflow end 126 of the frame 100, the biasing member 138 becomes axially compressed and positioned in a loaded state. While in the loaded state, the end of the biasing member 138 adjacent the flange 142 applies an axially-directed force in the direction of the inflow end 124 of the frame 100 to the locking member 136. In this way, the biasing member 138 can be configured, for example, to bias the locking member 136 in a first direction, such as toward the inflow end 124 of the frame 100 under the force of the biasing member and allow the locking member 136 to move axially in a second direction, opposite the first direction, such as toward the outflow end 126 of the frame 100 against the force of the biasing member. As shown in FIGS. 4 and 5, the biasing member 138 can also be coaxially disposed around the body 140 of the locking member 136.

The locking mechanism 104, as previously mentioned, can be positioned between a pair of adjacent inner and outer struts 118, 120 when the frame 100 is in a radially compressed state and an expanded state. As shown in FIGS. 3-5, for example, each locking mechanism 104 can generally be disposed between a first, radially inner strut 118 and a second, radially outer strut 120 which is adjacent the inner strut 118. More particularly, each locking mechanism 104 can be disposed between respective segments of the inner and outer struts 118, 120 which overlap and are pivotably coupled to one another at a common junction 116. The common junction 116, for instance, can be a junction corresponding to the location where an outer member 106 of a respective expansion mechanism 102 is secured to the frame 100, such as via fastener 128.

Each strut 118, 120 can form one of a plurality of outflow apices 146 of the frame 100. For instance, the inner strut 118 can be one of a pair of adjacent struts forming a first outflow apex 146a and the outer strut 120 can be one of a pair of adjacent struts forming a second outflow apex 146b. In this respect, the locking mechanism 104 and inner and outer struts 118, 120 are located at or proximate the outflow end 126 of the frame 100 and each locking mechanism 104 can be said to be disposed between a pair of adjacent outflow apices 146.

In some examples, rather than be located at the outflow end 126 and between an adjacent pair of outflow apices 146, the locking mechanism 104 can be located within a cell of the frame formed by inner and outer struts 118, 120. In such examples, the locking mechanism 104 can be disposed adjacent a junction 116 that is proximate the inflow end 124 of the frame. It should be appreciated that the locking mechanism 104 can be disposed adjacent any junction 116 along the length of the frame.

In some examples, multiple locking mechanisms 104 can be disposed along the length of a single expansion mechanism 102 (such as along the outer member 106) with each locking mechanism 104 being adjacent a corresponding junction 116. In such examples, the strut portions at each junction 116 having a locking mechanism 104 can include first and second locking features (described below) to cooperate with the locking mechanism 104 to retain the frame 100 in an expanded state.

As shown in FIGS. 3 and 4, the inner strut 118 can comprise a first or inner circumferentially extending locking feature 148 and the outer strut 120 can comprise a second or outer circumferentially extending locking feature 150. Specifically, as depicted in FIG. 4, the inner locking feature 148 can extend circumferentially from a longitudinal side edge 152 of the inner strut 118 and directed generally in the direction of the outer locking feature 150. Likewise, the outer locking feature 150 can extend circumferentially from a longitudinal side edge 154 of the outer strut 120 and directed generally in the direction of the inner locking feature 148. Each locking feature 148, 150 can have a radial thickness equal to the radial thickness of its respective strut 118, 120 (see FIG. 5).

Because each locking feature 148, 150 extends circumferentially, each inner and outer locking feature 148, 150 lies within the same plane as their respective strut 118, 120 and the two locking features 148, 150 radially overlap when the frame 100 in a radially compressed state (see FIG. 6). For instance, the inner locking feature 148 lies within same plane as the inner strut 118 and the second locking feature 150 lies within the same plane as the outer strut 120 and parallel to the plane of the inner strut 118 and locking feature 148. As such, the inner and outer locking features 148, 150 overlap as the inner and outer struts 118, 120 move toward one another when the frame 100 is radially compressed. In some instances, the inner and outer locking features 148, 150 can also partially overlap when the frame 100 is radially expanded, such as in a relatively lesser radially expanded state.

The biasing member 138, as previously mentioned, is configured to bias the locking member 136 of the locking mechanism 104 toward to the inflow end 124 of the frame 100. Accordingly, as the frame 100 is radially expanded, the locking member 136 of the locking mechanism 104 moves between and engages the inner and outer locking features 148, 150 to retain the frame 100 in a radially expanded state. Particularly, when the frame 100 is in a radially compressed configuration, the biasing member 138 can be in a loaded state and bias the locking member 136 toward the inflow end 124 and the common junction 116 of the inner and outer struts 118, 120 in such a way that the wedge member 144 abuts an end portion 156 of one or both of the locking features as they radially overlap (see FIG. 7). In this configuration, the locking member 136 can be said to be in a disengaged or unlocked state or position.

As the frame 100 expands, the inner and outer struts 118, 120 pivot relative to and away from one another, causing circumferential separation between the inner locking feature 148 and outer locking feature 150. The biasing member 138 continues to bias the locking member 136 toward the inflow end 124 and against the end portion 156 of the locking features 148, 150 until the separation between the locking features 148, 150 is enough to accommodate the wedge member 144 to extend therebetween. At which point, the biasing member 138 biases the wedge member 144 between and against inner edges 158 of the locking features 148, 150, as shown in FIGS. 3-4 and 6. In this configuration, the locking member 136 can be said to be in an engaged or locked state or position.

Engagement of the wedge member 144 with the inner and outer locking features 148, 150 retains the frame 100 in a locked configuration, where the frame 100 can be further radially expanded but not radially collapsed. In other words, the engagement between the wedge member 144 and inner and outer locking features 148, 150 permits pivoting movement of the struts 118, 120 relative to each other in a first direction to expand the frame 100 and resists pivoting movement of the struts 118, 120 relative to each other in a second direction to resist radial compression of the frame 100.

As one example, as the frame 100 is initially expanded and the locking member 136 begins to extend between and engage the locking features 148, 150, the wedge member 144 makes initial contact with the inner edges 158 of the locking features 148, 150. This engagement between the wedge member 144 and the locking features 148, 150 can retain or lock the frame 100 in this first radially expanded state while resisting radial compression of the frame 100. If the frame 100 is expanded beyond this first radially expanded state, the separation between the struts 118, 120 increases as the diameter of the frame 100 increases and the wedge member 144 extends and wedges further between the inner and outer locking features 148, 150 as the wedge member 144 continues to be biased axially toward the junction 116 of the inner and outer struts 118, 120. As such, the locking mechanism 104 can retain the frame 100 in a number of further radially expanded states as the wedge member 144 progressively moves closer toward the junction 116 as the frame 100 is expanded. That it is to say, the locking mechanism 104 is configured to retain the frame 100 in successive radially expanded states as the frame 100 is progressively expanded, such as in two or more radially expanded states. The locking mechanism 104 in this way, can retain the frame 100 in multiple radially expanded states along incremental expansion and/or along a continuous range of expansion.

The frictional engagement between the wedge member 144 of the locking member 136 and the inner and outer locking features 148, 150 can also prevent the wedge member 144 from being displaced relative to the locking features. Once the prosthetic valve is implanted within a selected implantation site, for instance, the patient's native anatomy (such as the native aortic annulus) can exert radial forces against a prosthetic heart valve and the frame 100 thereof, that tends to compress the frame 100. The position and frictional engagement of the wedge member 144 with the inner and outer locking features 148, 150 prevents such forces from displacing the locking member from between the locking features and toward the outflow end 126.

If disengaging the locking mechanism 104 is desired, such as to reposition or recapture and remove a prosthetic valve comprising the frame 100, the frame 100 can be unlocked by retracting the locking member 136 proximally until the wedge member 144 no longer engages the inner and outer locking features 148, 150. This can allow the struts 118, 120 to pivot freely relative to one another in either direction. In order to retract the locking member 136, as shown in FIGS. 3 and 4, a release member 176 can be coupled to the body 140 of the locking member 136 and configured to apply a proximally-directed force to the locking member 136 which is greater than the force the biasing member 138 applies to the wedge member 144. The release member 176 can be a rod, screw, cord, suture, cable, or the like, having a proximal end portion coupled to a handle of a delivery apparatus (see FIG. 9) which allows an operator to actuate and disengage the locking member 136 from the locking features 148, 150. In some examples, the distal end portion of the release member 176 can be releasably coupled to the body 140, such as via a threaded connection between the release member 176 and the body 140. For example, the distal end portion of the release member 176 can comprise a male threaded element that is inserted into a female threaded portion of the collar 143 (such as an internally threaded bore formed within the collar 143). Once the frame 100 is expanded to a desired radially expanded state, each release member 176 can be released or decoupled from its respective locking member 136 via a delivery apparatus and removed from the vasculature of the patient, such as by unscrewing the release members 176 from the collars 143.

As shown in FIGS. 3-5, the wedge member 144 of the locking mechanism 104 can be sized and shaped to facilitate proper engagement with the inner and outer locking features 148, 150. For instance, the wedge member 144 can be rectangular in shape and sized such that the wedge member 144 extends between the inner and outer locking features 148, 150 and spans a radial thickness T1 of the inner and outer struts 118, 120. As shown in FIG. 5 in particular, which depicts a magnified, side view of the locking mechanism 104 disposed between the inner and outer struts 118, 120, the wedge member 144 can extend between the inner and outer locking features 148, 150 from an inner surface 160 of the frame 100 to the outer surface 162 of the frame. In this way, the wedge member 144 can intersect a first plane including the inner strut 118 and inner locking feature 148, and a second plane including the outer strut 120 and the outer locking feature 150. By spanning the entirety of the radial thickness T1 of the inner and outer locking features 148, 150, the surface area of the wedge member 144 engaged with one or more locations along the inner edges 158 of the locking features 148, 150 is relatively greater than if the wedge member 144 were only to span a portion of the same radial thickness.

FIGS. 3 and 4 also show the inner and outer locking features 148, 150 can each comprise a plurality of outwardly extending teeth 178a, 178b, and 178c configured to engage the wedge member 144 during retention of the frame 100. Each individual tooth 178, for example, can have a respective apex which contacts the surface of the wedge member 144 during engagement with the inner and outer locking features 148, 150. Each locking feature 148, 150 can be integrally formed with its respective strut 118, 120, or can be coupled thereto.

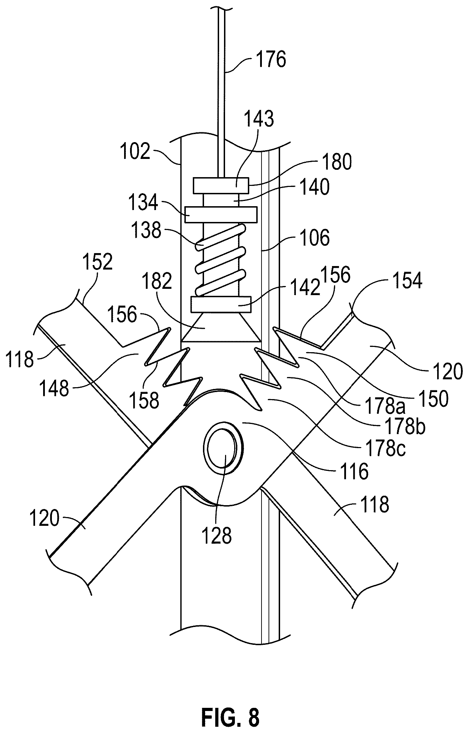

In some examples, the wedge member and/or the inner and outer locking features can have different configurations. For instance, two further examples of locking mechanisms are illustrated in FIGS. 6 and 8, respectively. In particular, FIG. 6 depicts a locking mechanism 164 according to one example and FIG. 8 depicts a locking mechanism 180 according to another example. The locking mechanism 164 and locking mechanism 180 can each include all of the components as previously described in connection with FIGS. 3-5 and locking mechanism 104.

FIG. 6 depicts the locking mechanism 164 in an unlocked state in which the locking mechanism abuts the end portion 156 of one or both of the inner and outer locking features 148, 150 when the frame 100 is in a radially compressed state. One difference between the locking mechanism 164 and locking mechanism 104, is that the locking mechanism 164 comprises a tapered wedge member 166 along one end of the body 140, wherein the wedge member 166 has a triangular cross-sectional profile in a plane parallel to the longitudinal axis of the wedge member. For instance, the wedge member 166 can taper in a longitudinal direction from the flange 142 of the locking mechanism 164 to a narrowed end or tip 168 directed toward the inflow end 124 of the frame 100 and strut junction 116.

In the illustrated example of FIG. 8, the locking mechanism 180 is shown retracted and/or otherwise disengaged from inner and outer locking features 148, 150 (such as via release member 176), such as to reposition or recapture the frame 100. A difference between the locking mechanism 180 and the locking mechanism 104 and locking mechanism 164, is that the locking mechanism 180 comprises a trapezoidal wedge member 182 along one end of the body 140. The wedge member 182 can, for example, have a trapezoidal cross-sectional profile parallel to the longitudinal axis of the wedge member. Accordingly and dissimilarly to the wedge member 166, the wedge member 182 can widen in a longitudinal direction from the flange 142 and toward the inflow end 124 of the frame 100 and a respective strut junction 116.

In a similar manner as the wedge member 144, the wedge member 166 and wedge member 182 can each span a radial thickness T1 of the inner and outer struts 118, 120 and their respective inner and outer locking features. Each of the wedge member 166 and wedge member 182 can, for example, have a radial thickness T1 along its respective longitudinal length (such as having a similar radial profile as wedge member 144 depicted in FIG. 5). In other examples, the wedge member 166 can be tapered and/or can be conical or frustoconical in shape.

In another instance, FIG. 7 shows a pair of inner and outer locking features 170, 172 according to another example, with the locking mechanism 164 extending between the locking features. Like the inner and outer locking features 148, 150, each inner and outer locking feature 170, 172 can extend circumferentially from a longitudinal side edge of a respective inner and outer strut 118, 120 and be equal in radial thickness to the struts. Rather than having a plurality outwardly extending teeth, however, the inner and outer locking features 170, 172 can each have a rounded edge 174 which curves radially outwardly from a longitudinal side edge 154 of a respective strut 118, 120. The rounded edges 174 can, in some examples, provide a continuous surface for the wedge member 166 (or wedge member 144) to engage, such as to allow the locking mechanism 164 (or locking mechanism 104) to retain the frame 100 in any expanded state within a range of expanded states.

Although described with some particularity, it should be appreciated that the wedge members and locking features described herein can have any number of various shapes, sizes, and configurations. For instance, the wedge members can also be textured and/or knurled to increase the friction between the wedge members and respective locking features.

FIG. 9 illustrates a delivery apparatus 200, according to one example, adapted to deliver a prosthetic heart valve 202, such as the illustrated prosthetic heart valve 10 or a prosthetic heart valve comprising frame 100 as described above. The prosthetic valve 202 can be releasably coupled to the delivery apparatus 200. It should be understood that the delivery apparatus 200 and other delivery apparatuses disclosed herein can be used to implant prosthetic devices other than prosthetic valves, such as stents or grafts.

The delivery apparatus 200 in the illustrated example generally includes a handle 204, a first elongated shaft 206 (which comprises an outer shaft in the illustrated example) extending distally from the handle 204, and at least one actuator assembly 208 extending distally through the outer shaft 206. The at least one actuator assembly 208 can be configured to radially expand and/or radially collapse the prosthetic valve 202 when actuated.

Though the illustrated example shows two actuator assemblies 208 for purposes of illustration, it should be understood that one actuator 208 can be provided for each actuator (such as each expansion mechanism 102) on the prosthetic valve. For instance, three actuator assemblies 208 can be provided for a prosthetic valve having three expansion mechanisms or actuators. In other examples, a greater or fewer number of actuator assemblies can be present.

In some examples, a distal end portion 216 of the shaft 206 can be sized to house the prosthetic valve in its radially compressed, delivery state during delivery of the prosthetic valve through the patient's vasculature. In this manner, the distal end portion 216 functions as a delivery sheath or capsule for the prosthetic valve during delivery,

The actuator assemblies 208 can be releasably coupled to the prosthetic valve 202. For instance, in the illustrated example, each actuator assembly 208 can be coupled to a respective actuator (such as expansion mechanism 102) of the prosthetic valve 202. For instance, each actuator assembly 208 can comprise an outer support sleeve 220 and an inner actuator member 222 extending coaxially through the support sleeve 220. As shown in FIG. 3, when used with a prosthetic valve having the frame 100, the inner actuator member 222 can be releasably coupled to an inner member 108 of an expansion mechanism 102, and the distal end of the support sleeve 220 can abut the proximal end of the outer member 106 of the expansion mechanism 102. When actuated, an actuator assembly 208 can transmit pushing and/or pulling forces to the outer and inner members 106, 108 of the expansion mechanism 102 of the prosthetic valve to radially expand and collapse the prosthetic valve as previously described. The actuator assemblies 208 can be at least partially disposed radially within, and extend axially through, one or more lumens of the outer shaft 206. For example, the actuator assemblies 208 can extend through a central lumen of the shaft 206 or through separate respective lumens formed in the shaft 206.

The handle 204 of the delivery apparatus 200 can include one or more control mechanisms (such as knobs or other actuating mechanisms) for controlling different components of the delivery apparatus 200 in order to expand and/or deploy the prosthetic valve 202. For instance, in the illustrated example the handle 204 comprises first, second, third and fourth knobs 210, 212, 214, and 215.

The first knob 210 can be a rotatable knob configured to produce axial movement of the outer shaft 206 relative to the prosthetic valve 202 in the distal and/or proximal directions in order to deploy the prosthetic valve from the delivery sheath 216 once the prosthetic valve has been advanced to a location at or adjacent the desired implantation location with the patient's body. For example, rotation of the first knob 210 in a first direction (for example, clockwise) can retract the sheath 216 proximally relative to the prosthetic valve 202 and rotation of the first knob 210 in a second direction (for example, counter-clockwise) can advance the sheath 216 distally. In other examples, the first knob 210 can be actuated by sliding or moving the knob 210 axially, such as pulling and/or pushing the knob.

In some examples, actuation of the first knob 210 (rotation or sliding movement of the knob 210) can produce axial movement of the actuator assemblies 208 (and therefore the prosthetic valve 202) relative to the delivery sheath 216 to advance the prosthetic valve distally from the sheath 216.

The second knob 212 can be a rotatable knob configured to produce radial expansion and/or contraction of the prosthetic valve 202. For instance, rotation of the second knob 212 in a first direction (for example, clockwise) can radially expand the prosthetic valve 202 (such as by moving the inner actuator members 222 proximally relative to the outer sleeves) and rotation of the second knob 212 in a second direction (for example, counterclockwise) can radially collapse the prosthetic valve 202 (such as by moving the inner actuator members 222 distally relative to the outer sleeves).

In some examples, the second knob 212 can be actuated by sliding or moving the knob 212 axially, such as pulling and/or pushing the knob. In other examples, the first and second actuators can each be coupled to a respective knob such that they can be actuated independently from one another.

The third knob 214 can be a rotatable knob configured to unlock the prosthetic heart valve 202 from its expanded configuration to radially compress the valve, such as for repositioning the prosthetic valve or withdrawing the prosthetic valve from the patient's vasculature. For example, the proximal end portions of the release members 176 can be operatively coupled to the third knob 214. Rotation of the knob 214 in one direction (for example, clockwise) can retract the release members 176, thereby retracting the locking members 136 and moving them to their unlocked state (such as shown in FIG. 6) to unlock the frame and allow for repositioning or recapture and removal. Rotation of the third knob in another direction (for example, counterclockwise) can release the locking members 136 and allow them to move to toward their locked state under the force of the biasing members 138 such that the locking members can extend between the locking features of the struts to resist radial compression of the frame and retain prosthetic valve in expanded state, as described herein.

In some examples, the third knob 214 can be actuated by sliding or moving the third knob 214 axially, such as pulling and/or pushing the knob.

As shown in the illustrated example, the handle 204 can include a fourth knob 215 (such as a rotatable knob) operatively connected to a proximal end portion of one or more of the actuators. The fourth knob 215 can be configured to, upon rotation of the knob, to decouple each of the one or more actuators from, for example, a respective component of the expansion mechanism (such as expansion mechanism 102) and/or locking mechanism (such as to decouple a release member 176 from a locking mechanism 104). For instance, the fourth knob 215 can be operable to rotate a first actuator in a direction that causes the first actuator to decouple from a respective expansion mechanism and/or respective locking mechanism. Similarly, the fourth knob 215 (or an additional knob) can be operable to rotate second and third actuators in a direction that causes the second and third actuators to decouple from respective expansion mechanisms and/or respective locking mechanisms. Once the actuators have been decoupled from the prosthetic valve, the delivery apparatus 200 can be removed from the patient.

### Delivery Techniques

For implanting a prosthetic valve within the native aortic valve via a transfemoral delivery approach, the prosthetic valve is mounted in a radially compressed state along the distal end portion of a delivery apparatus. The prosthetic valve and the distal end portion of the delivery apparatus are inserted into a femoral artery and are advanced into and through the descending aorta, around the aortic arch, and through the ascending aorta. The prosthetic valve is positioned within the native aortic valve and radially expanded (such as by inflating a balloon, actuating one or more actuators of the delivery apparatus, or deploying the prosthetic valve from a sheath to allow the prosthetic valve to self-expand). Alternatively, a prosthetic valve can be implanted within the native aortic valve in a transapical procedure, whereby the prosthetic valve (on the distal end portion of the delivery apparatus) is introduced into the left ventricle through a surgical opening in the chest and the apex of the heart and the prosthetic valve is positioned within the native aortic valve. Alternatively, in a transaortic procedure, a prosthetic valve (on the distal end portion of the delivery apparatus) are introduced into the aorta through a surgical incision in the ascending aorta, such as through a partial J-sternotomy or right parasternal mini-thoracotomy, and then advanced through the ascending aorta toward the native aortic valve.

For implanting a prosthetic valve within the native mitral valve via a transseptal delivery approach, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, into the right atrium, across the atrial septum (through a puncture made in the atrial septum), into the left atrium, and toward the native mitral valve. Alternatively, a prosthetic valve can be implanted within the native mitral valve in a transapical procedure, whereby the prosthetic valve (on the distal end portion of the delivery apparatus) is introduced into the left ventricle through a surgical opening in the chest and the apex of the heart and the prosthetic valve is positioned within the native mitral valve.

For implanting a prosthetic valve within the native tricuspid valve, the prosthetic valve is mounted in a radially compressed state along the distal end portion of a delivery apparatus. The prosthetic valve and the distal end portion of the delivery apparatus are inserted into a femoral vein and are advanced into and through the inferior vena cava, and into the right atrium, and the prosthetic valve is positioned within the native tricuspid valve. A similar approach can be used for implanting the prosthetic valve within the native pulmonary valve or the pulmonary artery, except that the prosthetic valve is advanced through the native tricuspid valve into the right ventricle and toward the pulmonary valve/pulmonary artery.

Another delivery approach is a transatrial approach whereby a prosthetic valve (on the distal end portion of the delivery apparatus) is inserted through an incision in the chest and an incision made through an atrial wall (of the right or left atrium) for accessing any of the native heart valves. Atrial delivery can also be made intravascularly, such as from a pulmonary vein. Still another delivery approach is a transventricular approach whereby a prosthetic valve (on the distal end portion of the delivery apparatus) is inserted through an incision in the chest and an incision made through the wall of the right ventricle (typically at or near the base of the heart) for implanting the prosthetic valve within the native tricuspid valve, the native pulmonary valve, or the pulmonary artery.

In all delivery approaches, the delivery apparatus can be advanced over a guidewire previously inserted into a patient's vasculature. Moreover, the disclosed delivery approaches are not intended to be limited. Any of the prosthetic valves disclosed herein can be implanted using any of various delivery procedures and delivery devices known in the art.

### Sterilization

Any of the systems, devices, apparatuses, etc. herein can be sterilized (for example, with heat/thermal, pressure, steam, radiation, and/or chemicals, etc.) to ensure they are safe for use with patients, and any of the methods herein can include sterilization of the associated system, device, apparatus, etc. as one of the steps of the method. Examples of heat/thermal sterilization include steam sterilization and autoclaving. Examples of radiation for use in sterilization include, without limitation, gamma radiation, ultra-violet radiation, and electron beam. Examples of chemicals for use in sterilization include, without limitation, ethylene oxide, hydrogen peroxide, peracetic acid, formaldehyde, and glutaraldehyde. Sterilization with hydrogen peroxide may be accomplished using hydrogen peroxide plasma, for example.

The treatment techniques, methods, steps, etc. described or suggested herein or in references incorporated herein can be performed on a living animal or on a non-living simulation, such as on a cadaver, cadaver heart, anthropomorphic ghost, simulator (e.g., with the body parts, tissue, etc. being simulated), etc.

### Additional Examples of the Disclosed Technology

In view of the above described implementations of the disclosed subject matter, this application discloses the additional examples enumerated below. It should be noted that one feature of an example in isolation or more than one feature of the example taken in combination and, optionally, in combination with one or more features of one or more further examples are further examples also falling within the disclosure of this application.

Example 1: An implantable device comprising: a radially expandable frame configured to move between a radially compressed state and a radially expanded state, the frame comprising a plurality of struts, at least one actuator mechanism configured to compress and expand the frame, and at least one locking mechanism coupled to the actuator mechanism; wherein a first strut and a second strut of the plurality of struts are pivotably coupled to one another at a common junction and configured to move toward one another as the frame is compressed and away from one another as the frame is expanded; wherein the first and second struts have first and second locking features that extend from longitudinal side edges of the first and second struts, respectively; wherein the locking mechanism comprises a locking member configured to extend between and engage the first and second locking features when the frame is expanded to retain the frame in the radially expanded state.

Example 2: The implantable device of any example herein, particularly example 1, wherein the locking member is configured to move between a locked position in which the locking member extends between and engages the first and second locking features and an unlocked position in which the locking member allows the first strut and the second strut to move toward one another and the frame to move from the expanded state into the compressed state.

Example 3: The implantable device of any example herein, particularly example 2, further comprising a release member releasably coupled to the locking member and configured to move the locking member from the locked position to the unlocked position.

Example 4: The implantable device of any example herein, particularly any one of examples 1-3, wherein the locking mechanism further comprises a biasing member configured to bias the locking member in a first direction to engage the first and second locking features.

Example 5: The implantable device of any example herein, particularly example 4, when combined with example 3, wherein the release member is configured to retract the locking member in a second direction opposite the first direction of the bias of the locking member to move the locking member to the unlocked position.

Example 6: The implantable device of any example herein, particularly any one of examples 1-5, wherein the locking member is configured to extend between and engage the first and second locking features when the frame is in a first radially expanded state and when the frame is in a second radially expanded state.

Example 7: The implantable device of any example herein, particularly example 6, wherein the frame has a first diameter in the first radially expanded state and a second diameter in the second radially expanded state.

Example 8: The implantable device of any example herein, particularly example 7, wherein the second diameter of the frame is greater than the first diameter of the frame.

Example 9: The implantable device of any example herein, particularly any one of examples 1-8, wherein the locking member extends radially from an inner surface of the frame to an outer surface of the frame.

Example 10: The implantable device of any example herein, particularly any one of examples 1-9, wherein the first and second locking features are directed in opposing circumferentially extending directions.

Example 11: The implantable device of any example herein, particularly any one of examples 1-10, wherein the first and second locking features are located proximate the common junction of the first and second struts.

Example 12: The implantable device of any example herein, particularly any one of examples 1-11, wherein the first and second locking features are located in an outflow region of the frame.

Example 13: The implantable device of any example herein, particularly any one of examples 1-12, wherein the first and second locking features are integrally formed with their respective struts.

Example 14: The implantable device of any example herein, particularly any one of examples 1-13, wherein the at least one actuator mechanism comprises a plurality of actuator mechanisms and the at least one locking mechanism comprises a plurality of locking mechanisms, each locking mechanism being coupled to a respective actuator mechanism.

Example 15: An implantable device comprising: a radially expandable frame configured to move between a radially compressed state and a radially expanded state, the frame comprising: a plurality of inner and outer struts forming an inflow end and an outflow end of the frame, an inner strut forming an inner circumferentially extending locking feature and an outer strut pivotably coupled to and adjacent the inner strut forming an outer circumferentially extending locking feature; at least one expansion mechanism configured to radially compress and expand the frame; and at least one locking mechanism coupled to the expansion mechanism and comprising a locking member configured to engage the inner and outer locking features of the inner and outer struts, wherein when the frame is in the radially compressed state, the inner and outer locking features overlap in a radial direction, and when the frame is in the radially expanded state, the inner and outer locking features are spaced circumferentially from one another and the locking member of the locking mechanism extends between and engages the inner and outer locking features to retain the frame in the radially expanded state.

Example 16: The implantable device of any example herein, particularly example 15, wherein the locking member is configured to span a radial thickness of the inner and outer locking features.

Example 17: The implantable device of any example herein, particularly example 15 or example 16, wherein the locking member is configured to span a radial thickness of the inner and outer struts.

Example 18: The implantable device of any example herein, particularly any one of examples 15-17, wherein a radial thickness of the inner and outer struts is equal to a radial thickness of the inner and outer locking features.

Example 19: The implantable device of any example herein, particularly any one of examples 15-18, wherein the inner locking feature extends circumferentially within a first plane and the outer locking feature extends circumferentially within a second plane parallel to the first plane.

Example 20: The implantable device of any example herein, particularly example 19, wherein the locking member intersects the first and second planes.

Example 21: The implantable device of any example herein, particularly any one of examples 15-20, wherein the inner locking feature is directed in a circumferential direction toward the outer locking feature and the outer locking feature is directed in a circumferential direction toward the inner locking feature.

Example 22: The implantable device of any example herein, particularly any one of examples 15-21, wherein the inner and outer locking features are proximate a pivot joint coupling the inner and outer struts.

Example 23: The implantable device of any example herein, particularly any one of examples 15-22, wherein the inner strut is one of a pair of adjacent struts forming a first outflow apex of the frame and the outer strut is one of a pair of adjacent struts forming a second outflow apex of the frame.

Example 24: The implantable device of any example herein, particularly any one of examples 15-23, wherein the inner and outer locking features are proximate the outflow end of the frame.

Example 25: The implantable device of any example herein, particularly any one of examples 15-24, the frame further comprising a plurality of inflow apices and outflow apices, wherein the locking member is disposed midway between a pair of adjacent outflow apices.

Example 26: The implantable device of any example herein, particularly any one of examples 15-25, wherein the expansion mechanism and locking member extend axially and parallel to a longitudinal axis of the frame.

Example 27: The implantable device of any example herein, particularly any one of examples 15-26, wherein the expansion mechanism and the locking member extend axially and parallel to one another.

Example 28: The implantable device of any example herein, particularly any one of examples 15-27, wherein when the frame is a first radially expanded state, the locking member engages corresponding first portions of the inner and outer locking features and when the frame is in a second radially expanded state, the locking member engages corresponding second portions.

Example 29: The implantable device of any example herein, particularly example 28, wherein when the locking member engages the first portions of the inner and outer locking features the frame has a first diameter, and when the locking member engages the second portions of the inner and outer locking features the frame has a second diameter greater than the first diameter.

Example 30: The implantable device of any of any example herein, particularly any one of examples 15-29, wherein the locking mechanism comprises a biasing member configured to bias the locking member to a locked position in which the locking member engages the locking features.

Example 31: The implantable device of any example herein, particularly example 30, wherein the biasing member biases the locking member in a direction toward the inflow end of the frame.

Example 32: The implantable device of any of any example herein, particularly any one of examples 15-31, further comprising a release member coupled to the locking mechanism and configured to disengage the locking member from the inner and outer locking features.

Example 33: An implantable device comprising: a radially expandable frame configured to move between a radially compressed state and a radially expanded state, the frame comprising: a plurality of inner and outer struts forming an inflow end and an outflow end, an inner strut having an inner locking feature circumferentially extending from a longitudinal side edge of the inner strut and an outer strut pivotably coupled to and adjacent the inner strut having an outer locking feature circumferentially extending from a longitudinal side edge of the outer strut; at least one expansion mechanism configured to compress and expand the frame; and a locking mechanism coupled to the expansion mechanism and comprising a wedge member configured to engage the inner and outer locking features and a biasing member configured to bias the wedge member between the inner and outer locking features as the frame is radially expanded; wherein the inner and outer struts are configured to move toward one another and overlap as the frame is radially compressed and move away from one another and circumferentially separate as the frame is radially expanded, wherein the wedge member engages an end portion of at least one of the inner and outer locking features when the frame is in the radially compressed state, and extends and wedges between the inner and outer locking features as the frame is radially expanded such that the inner and outer struts and frame are retained in the radially expanded state.

Example 34: The implantable device of any example herein, particularly example 33, wherein as the frame is continually expanded, the wedge member extends and wedges further between the inner and outer locking features such that the frame is retained in a relatively greater radially expanded state.

Example 35: The implantable device of any example herein, particularly example 33 or example 34, wherein the locking mechanism is configured to retain the frame in two or more successive radially expanded states.

Example 36: The implantable device of any example herein, particularly example 35, wherein a diameter of the frame progressively increases with each successive radially expanded state.

Example 37: The implantable device of any of any example herein, particularly any one of examples 33-36, wherein the wedge member and biasing member are coaxially aligned.

Example 38: The implantable device of any of any example herein, particularly any one of examples 33-37, the locking mechanism further comprising a support member extending outwardly from the expansion mechanism and coupled to the wedge member, the biasing member being situated between the support member and the wedge member.

Example 39: The implantable device of any example herein, particularly example 38, wherein the support member is perpendicular to the expansion mechanism.

Example 40: The implantable device of any example herein, particularly any one of examples 33-38, wherein the inner and outer locking features each comprise one or more circumferentially extending teeth.

Example 41. The implantable device of any example herein, particularly any one of examples 33-38, wherein the inner and outer locking features each comprise a circumferentially extending rounded edge.

Example 42: The implantable device of any example herein, particularly any one of examples 33-41, wherein the wedge member has a tapered profile.

Example 43: The implantable device of any example herein, particularly any one of examples 33-41, wherein the wedge member is rectangular in shape.

Example 44: The implantable device of any example herein, particularly any one of examples 33-41, wherein the wedge member has a trapezoidal profile.

Example 45: The implantable device of any example herein, particularly any one of examples 33-44, wherein the inner strut is one of a pair of struts forming a first outflow apex and the outer strut is one of another pair of struts forming a second outflow apex.

Example 46: The implantable device of any example herein, particularly example 45, wherein the wedge member and biasing member are disposed between the first and second outflow apices.

Example 47: The implantable device of any example herein, particularly any one of examples 33-46, wherein the wedge member is directed toward the inflow end of the frame.

Example 48: The implantable device of any example herein, particularly any one of examples 1-47, wherein the implantable device is a prosthetic valve comprising a valvular structure disposed inside a frame, wherein the valvular structure regulates the flow of blood through the frame in one direction.

Example 49: A method comprising: inserting a distal end of a delivery apparatus into a vasculature of a patient, the delivery apparatus releasably coupled to a prosthetic heart valve comprising a radially expandable frame configured to move between a radially compressed state and a radially expanded state, the frame comprising a plurality of struts, at least one expansion mechanism configured to compress and expand the frame; and at least one locking mechanism coupled to the expansion mechanism and comprising a locking member, wherein a first strut comprises a first circumferentially extending locking feature and a second strut coupled to and adjacent the first strut comprises a second circumferentially extending locking feature; advancing the prosthetic heart valve to a selected implantation location; expanding the frame of the prosthetic heart valve; and wedging the locking member of the locking mechanism between the first and second locking features, wherein engagement between the locking member and the first and second locking features prevents the struts from moving in a first direction to compress the frame and permits the struts to move in a second direction radially expand the frame.

Example 50: The method of any example herein, particularly example 49, wherein the first locking feature extends circumferentially within a first plane and the second locking feature extends circumferentially within a second plane parallel to the first plane as the frame is radially expanded.

Example 51: The method of any example herein, particularly example 49 or example 50, wherein the first locking feature is directed in a first direction and the second locking feature is directed in a second direction opposite the first direction.

Example 52: The method of any example herein, particularly any one of examples 49-51, wherein the first and second locking features are located in an outflow region of the frame.

Example 53: An implantable device or prosthetic valve of any example herein, particularly any one of examples 1-52, wherein the implantable device or prosthetic valve is sterilized.

The features described herein with regard to any example can be combined with other features described in any one or more of the other examples, unless otherwise stated. For example, any one or more of the features of one locking mechanism can be combined with any one or more features of another locking mechanism.

In view of the many possible ways in which the principles of the disclosure may be applied, it should be recognized that the illustrated configurations depict examples of the disclosed technology and should not be taken as limiting the scope of the disclosure nor the claims. Rather, the scope of the claimed subject matter is defined by the following claims and their equivalents.

The application further comprises the following embodiments:
1. An implantable device comprising:
   a radially expandable frame configured to move between a radially compressed state and a radially expanded state, the frame comprising a plurality of struts, at least one actuator mechanism configured to compress and expand the frame, and at least one locking mechanism coupled to the actuator mechanism;
   wherein a first strut and a second strut of the plurality of struts are pivotably coupled to one another at a common junction and configured to move toward one another as the frame is compressed and away from one another as the frame is expanded;
   wherein the first and second struts have first and second locking features that extend from longitudinal side edges of the first and second struts, respectively;
   wherein the locking mechanism comprises a locking member configured to extend between and engage the first and second locking features when the frame is expanded to retain the frame in the radially expanded state.
2. The implantable device of embodiment 1, wherein the locking member is configured to move between a locked position in which the locking member extends between and engages the first and second locking features and an unlocked position in which the locking member allows the first strut and the second strut to move toward one another and the frame to move from the expanded state into the compressed state.
3. The implantable device of embodiment 2, further comprising a release member releasably coupled to the locking member and configured to move the locking member from the locked position to the unlocked position.
4. The implantable device of any of embodiments 1-3, wherein the locking mechanism further comprises a biasing member configured to bias the locking member in a first direction to engage the first and second locking features.
5. The implantable device of embodiment 4, when depending on embodiment 3, wherein the release member is configured to retract the locking member in a second direction opposite the first direction of the bias of the locking member to move the locking member to the unlocked position.
6. The implantable device of any of embodiments 1-5, wherein the locking member is configured to extend between and engage the first and second locking features when the frame is in a first radially expanded state and when the frame is in a second radially expanded state.
7. The implantable device of embodiment 6, wherein the frame has a first diameter in the first radially expanded state and a second diameter in the second radially expanded state.
8. The implantable device of embodiment 7, wherein the second diameter of the frame is greater than the first diameter of the frame.
9. The implantable device of any of embodiments 1-8, wherein the locking member extends radially from an inner surface of the frame to an outer surface of the frame.
10. The implantable device of any of embodiments 1-9, wherein the first and second locking features are located proximate the common junction of the first and second struts.
11. The implantable device of any of embodiments 1-10, wherein the first and second locking features are integrally formed with their respective struts.
12. The implantable device of any of embodiments 1-11, wherein the at least one actuator mechanism comprises a plurality of actuator mechanisms and the at least one locking mechanism comprises a plurality of locking mechanisms, each locking mechanism being coupled to a respective actuator mechanism.
13. An implantable device comprising:
   a radially expandable frame configured to move between a radially compressed state and a radially expanded state, the frame comprising:
   a plurality of inner and outer struts forming an inflow end and an outflow end, an inner strut having an inner locking feature circumferentially extending from a longitudinal side edge of the inner strut and an outer strut pivotably coupled to and adjacent the inner strut having an outer locking feature circumferentially extending from a longitudinal side edge of the outer strut;
   at least one expansion mechanism configured to compress and expand the frame; and
   a locking mechanism coupled to the expansion mechanism and comprising a wedge member configured to engage the inner and outer locking features and a biasing member configured to bias the wedge member between the inner and outer locking features as the frame is radially expanded;
   wherein the inner and outer struts are configured to move toward one another and overlap as the frame is radially compressed and move away from one another and circumferentially separate as the frame is radially expanded,
   wherein the wedge member engages an end portion of at least one of the inner and outer locking features when the frame is in the radially compressed state, and extends and wedges between the inner and outer locking features as the frame is radially expanded such that the inner and outer struts and frame are retained in the radially expanded state.
14. The implantable device of embodiment 13, wherein as the frame is continually expanded, the wedge member extends and wedges further between the inner and outer locking features such that the frame is retained in a relatively greater radially expanded state.
15. The implantable device of embodiment 13 or embodiment 14, wherein the locking mechanism is configured to retain the frame in two or more successive radially expanded states.
16. The implantable device of embodiment 15, wherein a diameter of the frame progressively increases with each successive radially expanded state.
17. The implantable device of any of embodiments 13-16, wherein the wedge member and biasing member are coaxially aligned.
18. The implantable device of any of embodiments 13-17, the locking mechanism further comprising a support member extending outwardly from the expansion mechanism and coupled to the wedge member, the biasing member being situated between the support member and the wedge member.
19. The implantable device of any of embodiments 13-18, wherein the inner and outer locking features each comprise one or more circumferentially extending teeth.
20. A method comprising:
   inserting a distal end of a delivery apparatus into a vasculature of a patient, the delivery apparatus releasably coupled to a prosthetic heart valve comprising a radially expandable frame configured to move between a radially compressed state and a radially expanded state, the frame comprising a plurality of struts, at least one expansion mechanism configured to compress and expand the frame; and at least one locking mechanism coupled to the expansion mechanism and comprising a locking member, wherein a first strut comprises a first circumferentially extending locking feature and a second strut coupled to and adjacent the first strut comprises a second circumferentially extending locking feature;
   advancing the prosthetic heart valve to a selected implantation location;
   expanding the frame of the prosthetic heart valve; and
   wedging the locking member of the locking mechanism between the first and second locking features, wherein engagement between the locking member and the first and second locking features prevents the struts from moving in a first direction to compress the frame and permits the struts to move in a second direction radially expand the frame.

## Claims

1. An implantable device (10; 100) comprising:
a radially expandable frame (12) configured to move between a radially compressed state and a radially expanded state, the frame (12) comprising a plurality of struts (24; 24a; 24b; 118; 120), at least one actuator mechanism (50;102) configured to compress and expand the frame (12), and at least one locking mechanism (104; 164; 180) coupled to the actuator mechanism (50; 102);
wherein a first strut (24a) and a second strut (24b) of the plurality of struts (24; 24a; 24b; 118; 120) are pivotably coupled to one another at a common junction (28; 116) and configured to move toward one another as the frame (12) is compressed and away from one another as the frame (12) is expanded;
wherein the first and second struts (24a; 24b) have first and second locking features (148; 150; 170; 172) that extend from longitudinal side edges (154) of the first and second struts (24a; 24b), respectively;
wherein the locking mechanism (104; 164; 180) comprises a locking member (136) configured to extend between and engage the first and second locking features (148; 150; 170; 172) when the frame (12) is expanded to retain the frame (12) in the radially expanded state; wherein the locking mechanism (104; 164; 180) can be configured to retain the frame (12) in two or more successive radially expanded states.

2. The implantable device (10; 100) of claim 1, wherein a diameter of the frame (12) progressively increases with each successive radially expanded state.

3. The implantable device (10; 100) of claim 1 or 2, wherein the locking member (136) is configured to move between a locked position in which the locking member (136) extends between and engages the first and second locking features (148; 150; 170; 172) and an unlocked position in which the locking member (136) allows the first strut (24a) and the second strut (24b) to move toward one another and the frame (12) to move from the expanded state into the compressed state.

4. The implantable device (10; 100) of claim 3, further comprising a release member (176) releasably coupled to the locking member (136) and configured to move the locking member (136) from the locked position to the unlocked position.

5. The implantable device (10; 100) of any of claims 1 to 4, wherein the locking mechanism (104; 164; 180) further comprises a biasing member (138) configured to bias the locking member (136) in a first direction to engage the first and second locking features (148; 150; 170; 172).

6. The implantable device (10; 100) of claim 5, when depending on claim 4, wherein the release member (176) is configured to retract the locking member (136) in a second direction opposite the first direction of the bias of the locking member (136) to move the locking member (136) to the unlocked position.

7. The implantable device (10; 100) of any of claims 1 to 6, wherein the locking member (136) is configured to extend between and engage the first and second locking features (148; 150; 170; 172) when the frame (12) is in a first radially expanded state and when the frame (12) is in a second radially expanded state.

8. The implantable device (10; 100) of claim 7, wherein the frame (12) has a first diameter in the first radially expanded state and a second diameter in the second radially expanded state.

9. The implantable device (10; 100) of claim 8, wherein the second diameter of the frame (12) is greater than the first diameter of the frame (12).

10. The implantable device (10; 100) of any of claims 1 to 9, wherein the locking member (136) extends radially from an inner surface of the frame (12) to an outer surface of the frame (12); and/or wherein the first and second locking features (148; 150; 170; 172) are located proximate the common junction (28; 116) of the first and second struts (24a; 24b).

11. The implantable device (10; 100) of any of claims 1 to 10, wherein the first and second locking features (148; 150; 170; 172) are integrally formed with their respective struts (24; 24a; 24b; 118; 120); and/or wherein the at least one actuator mechanism (50; 102) comprises a plurality of actuator mechanisms (50; 102) and the at least one locking mechanism (104; 164; 180) comprises a plurality of locking mechanisms (104; 164; 180), each locking mechanism (104; 164; 180) being coupled to a respective actuator mechanism (50; 102).

12. The implantable device of any of claims 1 to 11, wherein
the plurality struts (24a; 24b) comprises a plurality of first struts (24a) and a plurality of second struts (24b), wherein the plurality of first struts (24a) and the plurality of second struts (24b) form an inflow end (14; 124) and an outflow end (16; 126),
wherein the locking member (136) comprises a wedge member (144) configured to engage the first and second locking features (148; 150; 170; 172) and a biasing member configured to bias the wedge member (144) between the first and second locking features (148; 150; 170; 172) as the frame (12) is radially expanded;
wherein the first and second struts (24a; 24b) are configured to move toward one another and overlap as the frame (12) is radially compressed and move away from one another and circumferentially separate as the frame (12) is radially expanded,
wherein the wedge member engages (144) an end portion of at least one of the first and second locking features (148; 150; 170; 172) when the frame (12) is in the radially compressed state, and extends and wedges between the first and second locking features (148; 150; 170; 172) as the frame (12) is radially expanded such that the first and second struts (24a; 24b) and frame (12) are retained in the radially expanded state.

13. The implantable device (10; 100) of claim 12, wherein as the frame (12) is continually expanded, the wedge member (144) extends and wedges further between the first and second locking features (148; 150; 170; 172) such that the frame (12) is retained in a relatively greater radially expanded state.

14. The implantable device (10; 100) of any of claims 11 to 13, wherein the wedge member (144) and biasing member (138) are coaxially aligned; and/or wherein the locking mechanism (104; 164; 180) further comprises a support member (134) extending outwardly from the expansion mechanism (50; 102) and coupled to the wedge member (144), the biasing member (138) being situated between the support member (134) and the wedge member (144).

15. The implantable device (10; 100) of any of claims 11 to 14, wherein the first and second locking features (148; 150; 170; 172) each comprise one or more circumferentially extending teeth (178; 178a; 178b; 178c).
